# FASCICULE DE BREVET EUROPEEN

(11) **EP 4 150 381 B1**
(45) Date de publication et mention de la délivrance du brevet: **01.07.2026**
(21) Numéro de dépôt: 21726092.6
(22) Date de dépôt: 12.05.2021
(51) Int. Cl.: G01V 3/08, G01R 19/00

(54) **PROCEDE DE DETECTION DANS UN MILIEU CONDUCTEUR DE L'ELECTRICITE**
DETEKTIONSVERFAHREN IN EINEM ELEKTRISCH LEITENDEN MEDIUM
METHOD OF DETECTION IN AN ELECTRICALLY CONDUCTIVE MEDIUM

(30) Priorité: 15.05.2020 FR 2004882
(43) Date de publication de la demande: 22.03.2023
(73) Titulaire: Elwave, 44470 Carquefou (FR); Institut Mines Telecom, 91120 Palaiseau (FR)
(72) Inventeur: DELPLANQUE, Quentin, Vincent, 44100 NANTES (FR); IFREK, Lyes, 44300 NANTES (FR); BOYER, Frédéric, 44850 MOUZEIL (FR); LEBASTARD, Vincent, Paul, Yannick, 44260 LA CHAPELLE LAUNAY (FR)
(74) Mandataire: Fidal Innovation
(86) Numéro de dépôt international: PCT/EP2021/062741
(87) Numéro de publication internationale: WO 2021/229010

(56) Documents cités:
- FR-A1- 2 694 333
- GB-A- 2 443 246
- QINGXUAN REN ET AL: "Amplitude information-frequency characteristics for multi-frequency excitation of underwater active electrolocation systems", BIOINSPIRATION & BIOMIMETICS, INSTITUTE OF PHYSICS PUBLISHING, BRISTOL, GB, vol. 15, no. 1, 19 November 2019 (2019-11-19), pages 16004, XP020350410, ISSN: 1748-3190, [retrieved on 20191119], DOI: 10.1088/1748-3190/AB526B
- JAMES R SOLBERG ET AL: "Active Electrolocation for Underwater Target Localization", INTERNATIONAL JOURNAL OF ROBOTICS RESEARCH, SAGE SCIENCE PRESS, THOUSAND OAKS, US, vol. 27, no. 5, 1 May 2008 (2008-05-01), pages 529 - 548, XP009156282, ISSN: 0278-3649, DOI: 10.1177/0278364908090538
- VON DER EMDE ET AL: "Biomimetic Sensors: Active Electrolocation of Weakly Electric Fish as a Model for Active Sensing in Technical Systems", JOURNAL OF BIONIC ENGINEERING, ELSEVIER, AMSTERDAM, NL, vol. 4, no. 2, 1 June 2007 (2007-06-01), pages 85 - 90, XP022856773, ISSN: 1672-6529, [retrieved on 20070601], DOI: 10.1016/S1672-6529(07)60018-3
- BOYER FREDERIC ET AL: "Underwater Reflex Navigation in Confined Environment Based on Electric Sense", IEEE TRANSACTIONS ON ROBOTICS, IEEE SERVICE CENTER, PISCATAWAY, NJ, US, vol. 29, no. 4, 1 August 2013 (2013-08-01), pages 945 - 956, XP011523506, ISSN: 1552-3098, [retrieved on 20130805], DOI: 10.1109/TRO.2013.2255451
- JIEGANG PENG: "A study of amplitude information-frequency characteristics for underwater active electrolocation system", BIOINSPIRATION & BIOMIMETICS, INSTITUTE OF PHYSICS PUBLISHING, BRISTOL, GB, vol. 10, no. 6, 4 November 2015 (2015-11-04), pages 66007, XP020293329, ISSN: 1748-3190, [retrieved on 20151104], DOI: 10.1088/1748-3190/10/6/066007

## Description

### DOMAINE DE L'INVENTION

La présente invention se rapporte à un procédé de détection dans un milieu conducteur de l'électricité.

### ARRIERE-PLAN TECHNOLOGIQUE

Plus précisément, l'invention se rapporte à un procédé de détection dans un milieu conducteur de l'électricité au moyen d'un système comportant une pluralité d'électrodes lui permettant de mettre en oeuvre le sens électrique, c'est-à-dire qu'un champ électrique est généré par certaines électrodes et que des mesures de grandeurs électriques liées à ce champ électrique sont exploitées pour obtenir des informations sur le milieu conducteur lui-même ou sur des objets se trouvant dans le milieu conducteur.

Dans le domaine de la détection dans un milieu conducteur de l'électricité, il est connu de mesurer des grandeurs électriques au moyen d'électrodes réceptrices pour en déduire des informations sur la présence d'objets ou bordures, ou des paramètres géométriques, tels que la forme de ces objets ou bordures, ainsi que leurs orientations, dans le milieu conducteur, sans connaissance a priori sur ces paramètres géométriques.

Par exemple, Ren et al., «Amplitude information-frequency characteristics for multi-frequency excitation of underwater active electrolocation systems», 2019, décrit un système d'électrolocalisation actif dans un milieu conducteur. Ren et al. enseigne un dispositif comportant une pluralité d'électrodes placées en contact direct avec le milieu (généralement deux électrodes d'émission et deux électrodes de réception) et utilise une excitation à plusieurs fréquences sinusoïdales afin d'améliorer la détection d'objets sous-marins. Le système mesure les grandeurs électriques (courant ou tension) aux électrodes réceptrices et applique un algorithme de reconnaissance (AIFC) pour extraire des informations sur la forme, la taille ou la conductivité de l'objet détecté. Par exemple, le document WO2013014392A1 décrit un procédé de contrôle du déplacement d'un système mobile dans un milieu conducteur d'électricité, le système comportant au moins une électrode en contact avec le milieu. Ce procédé comporte notamment une étape de mesure d'une caractéristique électrique - et plus précisément d'une intensité de courant électrique - du milieu par cette électrode, dite alors réceptrice. Dans un mode de réalisation particulier, le procédé de contrôle prévoit une étape de gestion, éventuellement automatisée, de la connectivité électrique des électrodes, qui peuvent être génératrices et/ou réceptrices dans le but d'optimiser la portée de détection ou la précision du positionnement du système mobile par rapport à un objet détecté.

Toutefois, le procédé décrit par WO2013014392A1 a pour but de gérer de manière optimale le déplacement d'un système mobile. La gestion de la connectivité électrique des électrodes est basée sur le déplacement du système mobile. Notamment, trois modes de connectivité sont définis : le mode attraction, qui permet de se rapprocher d'un objet, le mode répulsion qui permet de s'en éloigner, et le mode suivi des frontières d'un objet qui permet de longer un objet. Il est aussi possible de configurer les électrodes dans un mode où la portée de détection est optimale.

Toutefois, la grandeur électrique mesurée (amplitude d'une tension électrique ou d'une intensité électrique, à une fréquence fixée) ainsi que les états possibles des électrodes (génératrice, émettrice ou connectée à une borne commune à plusieurs électrodes (appelée dans ce document borne B1)) ne permettent pas d'obtenir des performances maximales pour des grandeurs d'intérêt pour la perception autres que la portée de détection ou la précision du positionnement du système mobile, telles que la forme, la taille ou la nature du matériau de l'objet détecté.

L'invention vise ainsi un procédé de détection dans un milieu conducteur d'électricité, éventuellement mis en 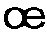 uvre indépendamment d'un système mobile, permettant d'optimiser de manière dynamique et automatique les performances d'un système de détection en termes, au choix, de portée de détection ou de localisation d'un objet ou de détermination de la forme d'un objet ou de la nature d'un objet.

### RESUME DE L'INVENTION

Ainsi, l'invention se rapporte à un procédé de détection dans un milieu conducteur d'électricité au moyen d'un système de détection selon la revendication 1.

Grâce à ces dispositions, il est possible de configurer de manière automatique et dynamique l'état des différentes électrodes de façon à ce que les performances de détection soient maximales par rapport à un objectif de détection déterminé avant chaque série de mesure. Par exemple, l'objectif peut être, à titre d'exemple, d'optimiser la portée de la détection dans une ou plusieurs directions de l'espace, ou encore la précision de la localisation d'un objet détecté, ou encore la précision de la reconnaissance de la forme et/ou de la nature de cet objet.

Dans un mode de réalisation, le procédé de détection comprend en outre une étape supplémentaire, dite étape d, au cours de laquelle le processeur calcule à partir des données de mesure au moins une donnée cartographique du milieu conducteur.

Grâce à cette disposition, le système de détection peut, à l'issue d'une série de mesure, fournir une carte de tout ou partie de l'espace qui l'environne. Cette carte peut ensuite être exploitée pour déplacer un système mobile.

Dans un mode de réalisation, les étapes du procédé sont répétées dans le même ordre au moins une fois, et en ce que des consignes sont transmises audit processeur pour contrôler la répétition des étapes soit avant la première étape a du procédé de détection par un opérateur distant ou non, soit au cours du procédé de détection par un opérateur distant.

Grâce à cette disposition, la carte fournit par le système de détection peut être enrichie par des informations complémentaires issues des séries de mesures successives et éventuellement adaptée en temps réelle. Cette carte peut ensuite être exploitée pour déplacer un système mobile, ou encore pour le suivre l'évolution de la **nature et/ou** de la position d'un ou plusieurs objets présents dans cet espace environnant.

Dans un mode de réalisation du procédé, le point de fonctionnement du système déterminé à l'étape a peut être choisi dans la liste {mode « portée », mode « localisation », mode « identification}, le mode « portée », permettant d'obtenir la portée de détection maximale dans une ou des directions données dudit milieu, le mode « localisation » permettant d'obtenir la précision maximale sur la localisation d'un objet antérieurement détecté, le mode « identification » permettant d'obtenir la meilleure résolution sur la forme et/ou la composition d'un objet antérieurement détecté.

Dans un mode de réalisation du procédé, le point de fonctionnement du système de détection passe automatiquement d'une étape a à la suivante :
- du mode « portée », s'il était dans ce mode, au mode « localisation » lorsqu'un objet est détecté et que cet objet est localisé à une distance inférieure à une distance seuil d2,
- du mode « localisation », s'il était dans ce mode, au mode « identification » lorsqu'un objet est détecté et que cet objet est localisé à une distance inférieure à une distance seuil d3 ou avec une forme et/ou une nature correspondant à une consigne,
- du mode « localisation », s'il était dans ce mode, au mode « portée» lorsqu'un objet est détecté et que la distance à laquelle se trouve un objet détecté devient supérieure à une distance seuil d2.

Ces dispositions permettent au système de détection de passer automatiquement du mode portée au mode localisation lorsqu'il se rapproche d'un objet détecté, puis au mode identification lorsqu'il s'en rapproche encore davantage, et enfin de revenir au mode portée s'il s'éloigne d'un objet après être passé dans le mode localisation.

Dans un mode de réalisation du procédé, la forme et/ou la fréquence et/ou l'amplitude du signal émis par les électrodes configurées dans l'état émettrice pour un point de fonctionnement déterminé à une étape a sont choisies à l'issue d'un balayage en fréquence.

Grâce à cette disposition, la fréquence de travail optimale pour la série de mesures à venir est déterminée sans connaissance a priori de l'espace environnant.

Dans un mode de réalisation du procédé, le signal émis par l'une au moins des électrodes configurées dans l'état émettrice pour un point de fonctionnement déterminé à une étape a est la composition d'au moins deux signaux sinusoïdaux de fréquences différentes.

Grâce à cette disposition, des informations correspondant à chacune des fréquences sont recueillies et permettent de détecter des éléments particuliers de l'espace environnant, comme, à titre d'exemple, une interface entre deux milieux différents.

Dans un mode de réalisation du procédé, si un objet est détecté à une étape d, l'amplitude et/ou la forme et/ou la fréquence des composantes sinusoïdales du signal électrique émis par chacune des électrodes configurées dans l'état émettrice pour un point de fonctionnement déterminé à une étape a ultérieure sont déterminées en fonction de la distance de l'objet détecté.

Cette disposition permet elle aussi de construire pas-à-pas une carte de l'espace environnant, ou encore de déplacer un système mobile de manière dynamique, c'est-à-dire en optimisant le point de fonctionnement de la série de mesures à venir en fonction des résultats de la détection de la dernière série de mesures effectuée.

Dans un mode de réalisation du procédé, si un objet est détecté à une étape d, les positions sur ledit système des électrodes configurées dans l'état émettrice pour un point de fonctionnement déterminé à une étape a ultérieure sont déterminées en fonction de la forme et/ou de la position d'un objet détecté.

Cette disposition permet de construire pas-à-pas une carte de l'espace environnant, ou encore de déplacer un système mobile de manière dynamique, c'est-à-dire en optimisant le point de fonctionnement de la série de mesures à venir en fonction des résultats de la détection de la dernière série de mesures effectuée.

Dans un mode de réalisation du procédé, des références connues sont utilisées pour déterminer l'au moins une donnée cartographique à l'étape d.

Grâce à cette disposition, on peut utiliser par exemple des abaques comprenant des signatures électriques d'objet pour conclure sur la nature des objets détectés.

Corrélativement, l'invention concerne un programme d'ordinateur selon la revendication 11.

L'invention concerne aussi un système de détection dans un milieu conducteur d'électricité selon la revendication 12.

Dans un mode de réalisation, le système mobile comporte en outre un module de contrôle adapté pour contrôler le déplacement dudit système mobile à partir des résultats de mesure du système de détection obtenues en suivant le procédé de détection dans un des modes de réalisation décrits plus haut.

Grâce à cette disposition, le système mobile peut se déplacer sans connaissance a priori du milieu dans lequel il évolue.

Dans un mode de réalisation du système mobile, les électrodes du système de détection qui l'équipe sont réparties sur au moins une partie de la surface dudit système mobile en contact avec ledit milieu.

### BREVE DESCRIPTION DES DESSINS

Des modes de réalisation de l'invention seront décrits ci-dessous par référence aux dessins, décrits brièvement ci-dessous :
Fig. 1 représente une réalisation d'un système mobile équipé d'un système de détection muni d'électrodes réparties au niveau de sa surface en contact avec le milieu extérieur.
Fig. 2 représente une architecture électrique d'un boîtier de commutation.
Fig. 3 représente en détail un exemple d'une cellule de commutation d'une électrode donnée.
Fig. 4 représente le circuit électrique des cellules correspondant aux électrodes i et j dans l'état connectée, lorsque l'électrode i est en mode de mesure U et l'électrode j en mode de mesure I.
Fig. 5 représente la proportion du temps pendant laquelle les électrodes sont activées, en fonction de leur position sur le système de détection, dans le cas simplifié d'un système de détection dans un milieu à deux dimensions en mode portée « isotrope ». Le système de détection est dans cet exemple particulier immobile dans le milieu conducteur.
Fig. 6 représente la proportion du temps pendant laquelle les électrodes sont activées, en fonction de leur position sur le système de détection, dans le cas simplifié d'un système de détection dans un milieu à deux dimensions, dans une réalisation particulière du mode portée « anisotrope ». Le système de détection est dans cet exemple particulier en translation rectiligne dans le milieu conducteur.
Fig. 7 représente la proportion du temps pendant laquelle les électrodes sont activées, en fonction de leur position sur le système de détection, dans le cas simplifié d'un système de détection dans un milieu à deux dimensions, dans une réalisation particulière du mode portée « anisotrope ». Le système de détection est dans cet exemple particulier en rotation autour de l'un de ses axes de symétrie.
Fig. 8 représente un algorithme permettant de réaliser le mode calibration.
Fig. 9 représente un algorithme permettant de réaliser le mode portée.
Fig. 10 représente un algorithme permettant de réaliser le mode localisation.
Fig. 11 représente un algorithme permettant de réaliser le mode identification.
Fig. 12 représente de manière schématique les différents étages du système dans le mode de réalisation « électronique centralisée ».
Fig. 13 représente de manière schématique les différents étages du système dans le mode de réalisation « électronique centralisée ».

Sur les dessins, des références identiques désignent des objets identiques ou similaires.

### DESCRIPTION DETAILLEE

Ainsi, l'invention porte sur un procédé de détection dans un milieu conducteur d'électricité au moyen d'un système de détection.

Le système de détection mettant en œuvre le procédé comprend une pluralité d'électrodes Ei destinées à être en contact électrique direct avec le milieu conducteur.

Par exemple, les électrodes Eᵢ peuvent être réparties au choix à la surface externe d'un système mobile 100 que le système de détection équipe, de sorte que les électrodes soient en contact électrique direct avec le milieu conducteur.

Le milieu conducteur est par exemple l'eau.

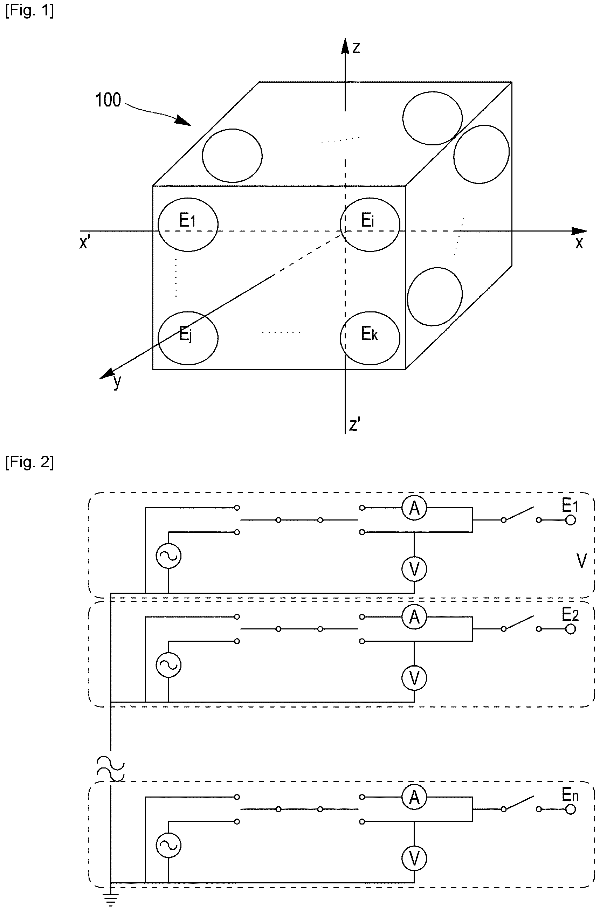

La figure 1 représente de façon schématique un système mobile 100 conforme à un mode particulier de réalisation de l'invention. Ce système mobile 100 comporte un corps parallélépipédique de dimensions de l'ordre de 1000 mm * 1000 mm * 1000 mm. Les trois axes de ce parallélépipède, centrés sur le centre O du parallélépipède, constituent un repère d'espace (Oxyz) pour le référentiel du système de détection.

Les n électrodes Eᵢ (i entier compris entre 1 et n) du système de détection peuvent être réparties à la surface du système mobile 100 de manière à être en contact avec le milieu conducteur d'électricité. Dans le mode de réalisation particulier décrit ici, la surface exposée au milieu conducteur d'une électrode Eᵢ donnée est un disque et 24 électrodes sont réparties aux coins et sur les arêtes du parallélépipède.

Ce mode de réalisation n'est pas limitatif. La répartition et/ou la forme des électrodes Eᵢ peut être adaptée à la géométrie du système mobile et du milieu conducteur à explorer. En particulier, il est possible de placer une partie des électrodes sur chacune des faces du parallélépipède.

Le système mobile 100 peut aussi ne pas être parallélépipédique. Il peut par exemple être cylindrique ou d'une forme quelconque.

Dans le cas où le système mobile 100 comporte plusieurs parties mobiles les unes par rapport aux autres, les électrodes Eᵢ peuvent être réparties sur l'ensemble de ces parties mobiles ou sur une fraction de celles-ci seulement.

Le nombre d'électrodes Eᵢ peut aussi être adapté aux dimensions du système mobile. Dans le cas d'un parallélépipède de dimension caractéristique de l'ordre de 1000 mm, 8 électrodes disposées aux huit sommets du parallélépipède permettent par exemple d'explorer l'ensemble des directions de l'espace entourant le parallélépipède sans laisser d'angle mort.

Les électrodes sont résistantes à la corrosion, par exemple en Inox 316, ou encore en platine, titane ou graphite, fibre de carbone, et disposées sur un support isolant électriquement tel que du polychlorure de vinyle.

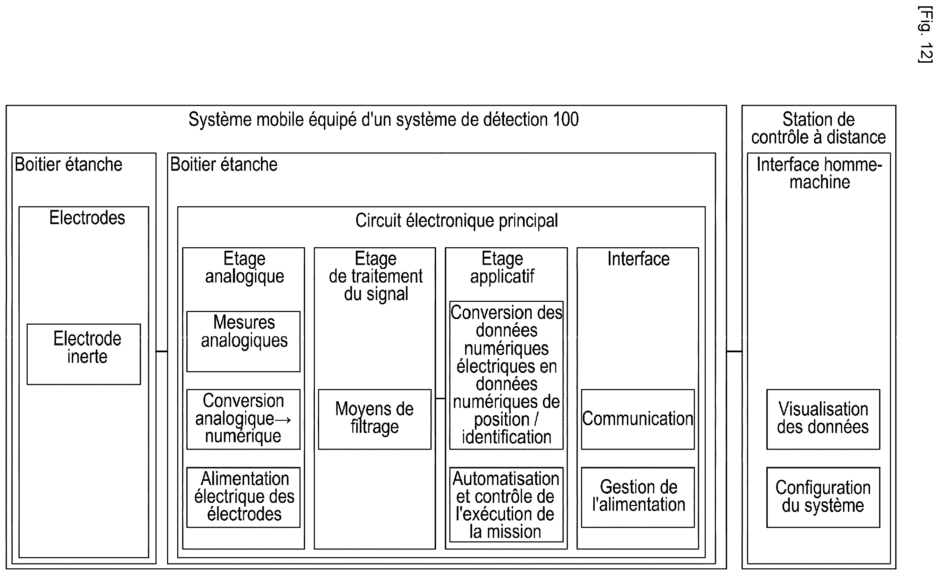

Dans le mode de réalisation « électronique centralisée » suivant la figure 12, les électrodes Eᵢ sont connectées électriquement par l'intermédiaire d'un faisceau de câbles souples à un boîtier étanche de dimensions de l'ordre de la dizaine de centimètres.

Dans le mode de réalisation décrit ici, ce boîtier contient :
- le bloc de génération de consigne comportant un microprocesseur, une mémoire morte comportant un programme d'ordinateur exécutable par le microprocesseur, une mémoire vive permettant l'exécution de ce programme, et des moyens de communication des consignes au boîtier de commutation et de réception des informations en provenance du boîtier de commutation ;
- le boîtier de commutation (ou équivalemment bloc de commutation), qui contient autant de cellules de commutation que d'électrodes, ainsi que les composants électroniques et électriques nécessaires pour réaliser le circuit électrique suivant la figure 2.

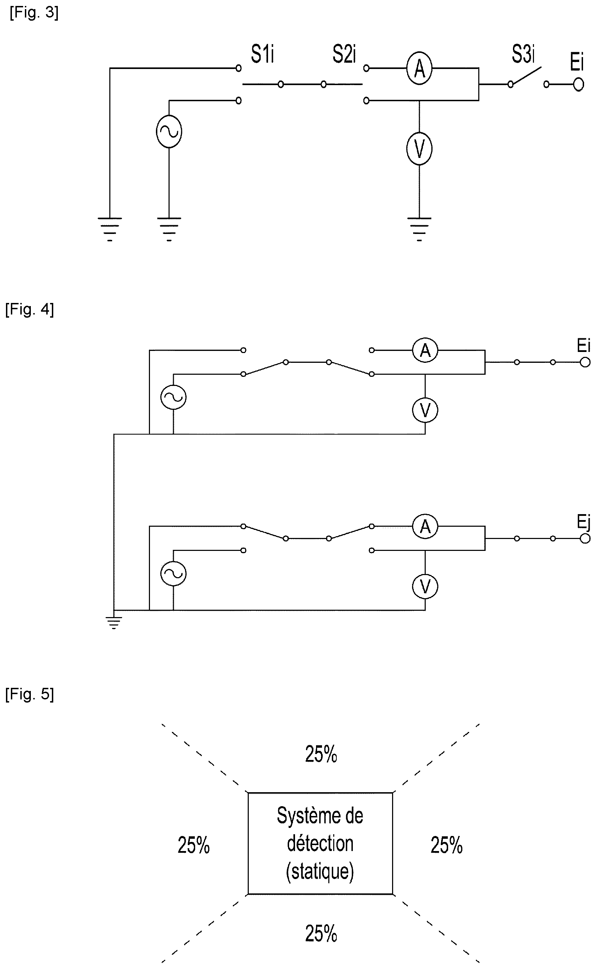

Chaque cellule de commutation est dédiée à une électrode et peut comporter un générateur par électrode, les moyens de mesure des grandeurs électriques, et les composants électriques et électroniques nécessaires pour réaliser le circuit électronique suivant la figure 3. Il est aussi possible qu'un générateur soit mutualisé entre plusieurs électrodes, voire la totalité des électrodes.

Ainsi, pour l'électrode Eᵢ, l'interrupteur S₃ᵢ permet de placer l'électrode i dans l'état de connexion connecté ou déconnecté. Si l'électrode est connectée, elle peut être placée dans l'état émettrice ou dans l'état réceptrice par l'intermédiaire de l'interrupteur S₁ᵢ.

Une électrode donnée Eᵢ peut donc être dans trois états différents : émettrice (et donc connectée), réceptrice (et donc connectée), déconnectée.

Dans ce cas, l'interrupteur S₂ᵢ place l'électrode dans le mode de mesure I ou le mode de mesure U définis ci-après.

Les interrupteurs S₁ᵢ, S₂ᵢ, S₃ᵢ peuvent être pilotés par le boîtier de génération de consignes, de sorte que l'état de chaque électrode peut être librement configuré à chaque étape du procédé.

Dans ce mode de réalisation, aucune intervention humaine n'est nécessaire au niveau du boîtier de commutation. La reconfiguration des électrodes peut donc se faire à distance, de manière automatique comme cela sera décrit plus loin. En particulier, lorsque le système de détection est immergé dans le milieu conducteur d'électricité, il est possible de reconfigurer les électrodes de manière automatique sans changer la position du système de détection ou du système mobile 100 que ce système de détection équipe.

Le boîtier étanche du système de détection peut être utilisé tel quel ou intégré à l'intérieur d'un système mobile 100 qu'il équipe, ou encore être placé sur la surface externe d'un tel système mobile 100.

Il peut par exemple être utilisé dans le cadre de la surveillance des vibrations de structures fixes comme des infrastructures d'extraction pétrolière.

Une électrode Eᵢ placée dans l'état émettrice est connectée à un générateur de tension approprié, de sorte que l'amplitude, la fréquence et/ou la forme du potentiel électrique de l'électrode peuvent être imposées au sein de la gamme permise par le générateur de tension.

Choisir l'amplitude du potentiel électrique d'un électrode placée dans l'état émettrice revient à choisir la puissance électrique fournie par le générateur connecté à cette électrode. Pour simplifier la formulation de, on pourrait équivalemment parler de « choisir la puissance du signal émis par une électrode émettrice ».

L'amplitude, la fréquence et/ou la forme du potentiel électrique de l'électrode peuvent être imposées pour chaque électrode placée dans l'état émettrice de manière indépendante des autres électrodes placées dans l'état émettrice.

Par exemple, un générateur peut être fourni pour chaque électrode.

Dans un autre mode de réalisation un même générateur peut être connecté à plusieurs électrodes placées dans l'état émettrice.

A titre d'exemple non limitatif, l'amplitude de ce potentiel électrique peut être choisie dans la gamme [ 0 V, 15 V ] et sa fréquence peut être choisie dans la gamme ] 0 Hz, 3 MHz]. La forme du potentiel peut être à titre d'exemple sinusoïdale, carrée, triangulaire. Le potentiel électrique peut être périodique, ou encore ne contenir qu'une ou plusieurs impulsions.

Dans le cas où plusieurs générateurs sont prévus, tous les générateurs connectés à des électrodes placées dans l'état émettrice sont activés simultanément.

Les électrodes émettrices génèrent chacune un champ électrique dans l'espace environnant. Une fraction des lignes de ces champs aboutissent sur les électrodes dans l'état réceptrice. Cette fraction dépend du dipôle électrode émettrice - électrode réceptrice considéré, c'est-à-dire des positions relatives des électrodes du couple d'électrodes émettrice et réceptrice considéré.

Les champs électriques générés par l'ensemble des électrodes se superposent pour former un champ électrique résultant dont la topographie dépend non seulement des positions et formes des électrodes émettrices et potentiels de ces électrodes, mais aussi des positions et formes des électrodes réceptrices (dont le potentiel est celui de la masse électrique), ainsi que des positions et formes des électrodes déconnectées.

Dans l'état déconnectée, une électrode Eᵢ n'est reliée électriquement à aucun élément du système de détection ou du système mobile. En particulier, deux électrodes Eᵢ différentes placées simultanément dans l'état déconnectée ne sont pas reliées entre elles. Une électrode Eᵢ déconnectée est à l'abandon, c'est-à-dire qu'elle prend le potentiel électrique du milieu avec lequel elle est en contact. Son potentiel électrique n'est pas imposé. L'électrode est libre de se polariser du fait de son environnement

Par ailleurs, l'état déconnectée rend impossible la circulation d'un courant électrique dans une électrode placée dans ce mode, puisqu'elle n'est intégrée à aucun circuit électrique fermé. L'existence de ce mode permet donc d'imposer les électrodes réceptrices qui seront effectivement traversées par un courant électrique.

L'existence de l'état déconnectée permet donc des points de vue possibles pour le système de détection plus nombreux qu'en l'absence de ce mode.

La variété des combinaisons possibles de types d'électrodes est l'un des facteurs qui rend possible l'optimisation du système de détection par rapport au but recherché.

La reconfiguration des électrodes, c'est-à-dire du point de fonctionnement du système, entre deux séries de mesures successives (la notion de série de mesure sera définie plus loin), ainsi que cela sera décrit plus loin, permet de faire varier d'une série de mesures à la suivante la topographie du champ électrique généré par le système de détection dans la scène environnante.

Un champ électrique anisotrope donnera par exemple des informations différentes sur la scène de celles obtenues avec un champ électrique isotrope. Deux champs électriques anisotropes de topographies différentes fourniront des informations différentes, et ce même si le système de détection n'a pas changé de position et/ou d'orientation.

L'existence de l'état déconnectée permet notamment d'explorer des directions particulières du milieu conducteur, en générant un champ électrique d'intensité significative essentiellement dans des directions particulières, fixées entre autres par les électrodes qui ne sont pas dans l'état « déconnectée».

L'existence de l'état déconnecté permet, même avec des électrodes réparties sur l'ensemble de la surface d'un système mobile tel que représenté sur la figure 1 de définir un champ électrique permettant la détection dont la topographie est très fortement anisotrope. Par exemple, il est possible de déconnecter toutes les électrodes sauf celles d'une face. Dans ce cas, seules les électrodes de cette dernière face pourront éventuellement être traversées par un courant électrique.

Une électrode configurée dans l'état réceptrice peut être configurée dans deux modes de mesure différents :
- le mode de mesure « I » : dans ce cas, le potentiel électrique de cette électrode est imposé et égal à celui de la masse électrique du système. La grandeur électrique mesurée est l'intensité du courant électrique circulant dans l'électrode réceptrice, c'est-à-dire que sa phase et son amplitude sont mesurées. Pour ce faire, N mesures de l'intensité sont effectuées pour chaque période du signal, pendant P périodes du signal. Si le signal comporte plusieurs composantes sinusoïdales, des étapes de filtrage de bandes passantes adaptées aux différentes composantes à étudier sont effectuées de manière à mesurer la phase et l'amplitude de chacune des composantes de l'intensité électrique circulant dans l'électrode réceptrice. La différence de potentiel entre l'électrode émettrice et l'électrode réceptrice mises en jeu est dans ce cas imposée donc connue, ce qui permet au final de déduire de la mesure une valeur d'impédance pour chaque fréquence étudiée.
- le mode de mesure « U » : dans ce cas, le potentiel de l'électrode réceptrice est flottant. L'intensité du courant électrique circulant dans les électrodes au niveau de l'électrode émettrice ainsi que le potentiel électrique au niveau de l'électrode réceptrice sont mesurés, c'est-à-dire que leurs phases et amplitudes respectives sont mesurées. Pour ce faire, N mesures de l'intensité (respectivement de la tension) sont effectuées pour chaque période du signal, pendant P périodes du signal. Si le signal comporte plusieurs composantes sinusoïdales, des étapes de filtrage au moyen de filtres électroniques de bandes passantes adaptées aux différentes composantes à étudier sont effectuées de manière à mesurer la phase et l'amplitude de chacune des composantes de l'intensité électrique circulant dans l'électrode émettrice (respectivement du potentiel électrique de l'électrode réceptrice).

L'étage de filtrage est placé à l'entrée du boîtier de génération de consigne de sorte que le filtrage des différentes composantes est effectué sur le signal numérique provenant des moyens de mesure après conversion analogique-numérique, avant que ces données ne soient traitées pour obtenir les informations sur la position, la nature ou la forme de l'objet détecté.

La figure 4 donne un exemple de point de fonctionnement pour lequel les électrodes Eᵢ et Eⱼ sont connectées, la première étant dans l'état émettrice et la seconde dans l'état réceptrice en mode de mesure I.

Chacune des N mesures nécessite une « unité de temps » caractéristique, qui dépend de l'électronique effectivement choisie pour réaliser le système de détection.

Une série de mesures prend fin dès lors que chacune des N*P mesures pour chacune des composantes du signal a été effectivement réalisées. Une série de mesure comprend donc l'évaluation d'au moins une grandeur électrique (parmi les grandeurs intensité électrique et potentiel électrique) au niveau de chacune des électrodes configurées dans l'état réceptrice ou émettrice.

Le point de fonctionnement du système est fixé avant chaque série de mesures par la configuration des électrodes, et plus précisément la configuration des trois paramètres suivants :
- l'état de chacune des électrodes, choisi parmi les au moins trois états : émettrice, réceptrice, déconnectée ;
- la fréquence d'une composante d'un signal électrique émis par au moins une des électrodes émettrices ;
- l'amplitude du signal électrique émis par au moins une des électrodes configurées dans l'état émettrice.

L'impédance caractéristique de la portion de milieu conducteur électrique entre l'électrode émettrice et l'électrode réceptrice d'un dipôle électrode réceptrice - électrode émettrice donné est alors déduite de la différence de potentiel entre ces deux électrodes et du courant circulant dans l'une d'elles pour chacune des fréquences de travail. Ces impédances peuvent ensuite être exploitées par le système de détection pour obtenir des paramètres caractérisant l'objet à détecter (ou l'absence d'objet).

On comprend donc que dans cette invention, la notion de détection comprend l'un et ou l'autre des deux aspects suivants : localisation et caractérisation d'un objet. On pourrait donc équivalemment parler de perception.

Lorsqu'une série de mesures est effectuée, autant d'impédances électriques que de dipôles électrode émettrice - électrode réceptrice constitués dans le point de fonctionnement choisi peuvent être évaluées pour chacune des fréquences de travail.

Le système de détection peut être asservi en amplitude pour protéger les électrodes : si l'intensité du courant détecté dans une des électrodes en mode de mesure I est supérieure à une valeur de consigne, la série de mesures en cours s'arrête et les amplitudes des tensions aux bornes des générateurs connectés aux électrodes dans l'état émettrice sont réduites pour le point de fonctionnement suivant.

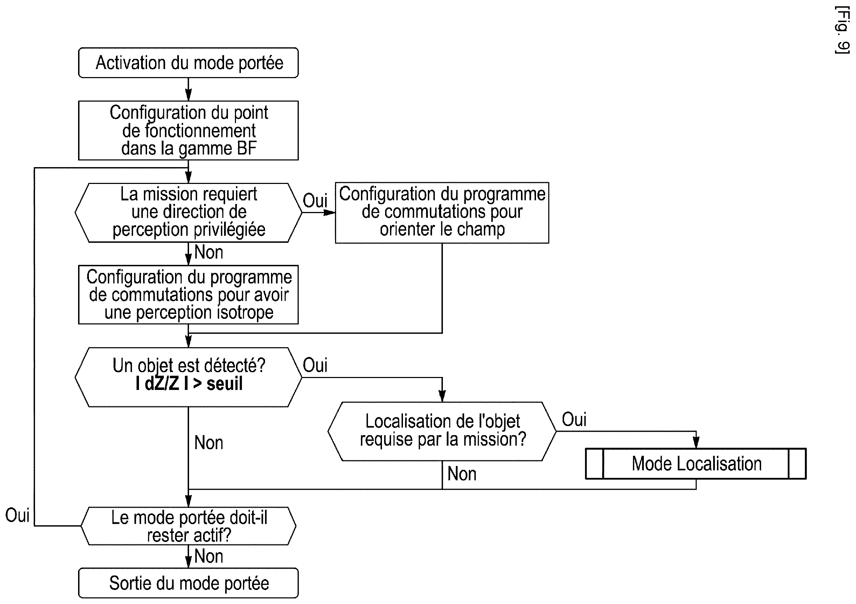

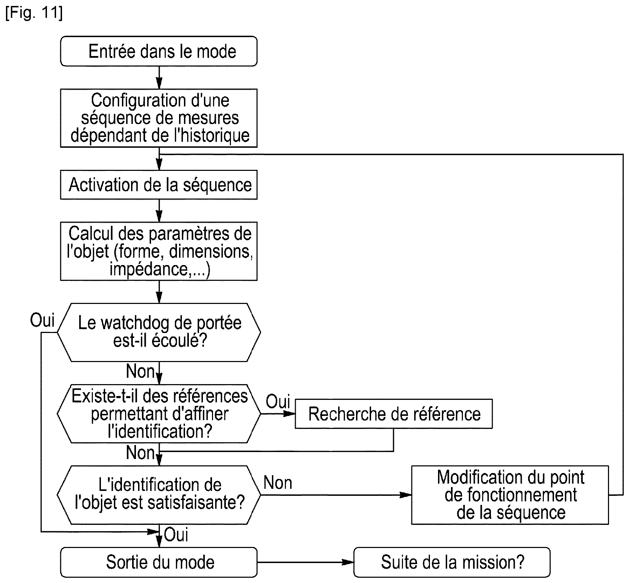

Les résultats sur la position et/ou la forme et/ou la nature d'un éventuel objet détecté, ou le fait qu'aucun objet n'est détecté sont enfin éventuellement utilisés par le boîtier de génération de consignes du système de détection pour déterminer le point de fonctionnement du système pour la série de mesures suivante, par exemple suivant les algorithmes représentés sur les figures 9 à 11.

Selon une réalisation, dans le procédé de détection, le point de fonctionnement du système peut être choisi pour qu'il corresponde à un mode « portée ». Dans ce cas, les groupes d'électrodes placées dans les états émettrice ou réceptrice sont déterminés en fonction des directions dans lesquelles on souhaite obtenir une portée de détection maximale, c'est-à-dire que la distance à laquelle un objet peut être détecté est maximale pour un fonctionnement nominal des composants électriques du circuit.

Dans une réalisation particulière du mode portée, représentée dans une version simplifiée à la figure 5, la répartition des électrodes dans chacun des trois états émettrice, réceptrice et déconnectée est « isotrope », c'est-à-dire aussi isotrope que possible étant donné les positions des électrodes Eᵢ du système de détection. Aucune des directions de l'espace n'est privilégiée dans cette répartition.

Par exemple, dans le cas où le système de détection équipe un système mobile 100 de la manière représentée sur la figure 1, des mesures en mode portée peuvent être répétées 6n fois. Pour chacune des six séries de n séries de mesures, seules des électrodes à l'intérieur, sur les arêtes et/ou sur les coins d'une seule à la fois des six faces du parallélépipède peuvent être placées dans l'état émettrice, les six faces étant balayées successivement.

La figure 5 représente ainsi dans un cas simplifié bidimensionnel le temps alloué à l'activation des électrodes sur chacune des quatre faces du système mobile rectangulaire simplifié équipé du système de détection.

Dans le mode portée, les amplitudes des tensions au niveau des électrodes émettrices peuvent être comprises dans la gamme [0, 15V]. La forme et la fréquence des tensions peuvent être les mêmes pour toutes ces électrodes. Une telle réalisation isotrope du mode portée permet par exemple d'explorer l'espace dans les 3 directions du référentiel du système mobile simultanément avec la même portée.

A l'inverse, si le système de détection a détecté un objet dans une direction particulière ou si une direction est d'intérêt particulier pour l'exploration, par exemple si un système mobile 100 que le système de détection équipe est en translation dans cette direction, le système de détection pourra être placé en mode portée avec une gestion « anisotrope » des électrodes configurées dans l'état connectée.

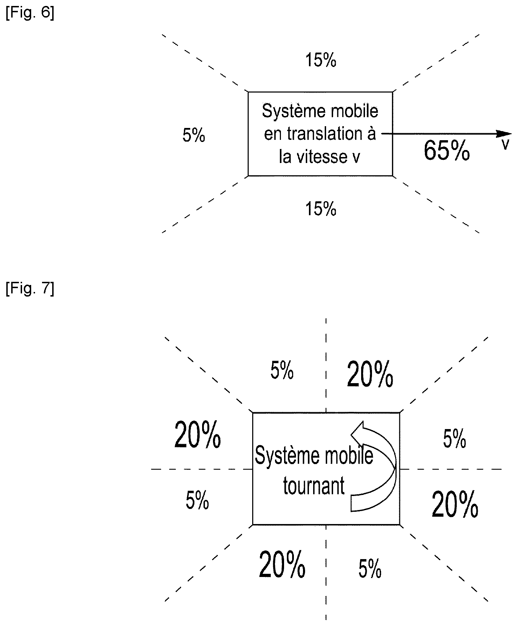

Dans l'exemple simplifié représenté à la figure 6, pour un système de détection équipant un système mobile bidimensionnel, si la direction d'intérêt est la direction [Ox), une fraction supérieure à 25% du nombre de séries de mesures (ici 65% à titre d'exemple) sera effectuée en n'activant des électrodes que à l'intérieur, sur les arêtes et/ou sur les coins de la face de normale sortante de même sens et direction que [Ox) et une fraction supérieure à la moitié des mesures restant (ici 15% à titre d'exemple) en n'activant des électrodes que à l'intérieur, sur les arêtes et/ou sur les coins de la face opposée.

Dans un cas tridimensionnel réel, n séries de mesure en mode portée seront effectuées, de sorte que plus d'un sixième des n séries de mesures sera effectué en n'activant des électrodes que à l'intérieur, sur les arêtes et/ou sur les coins de la face de normale sortante de même sens et direction que [Ox) et une fraction supérieure à un cinquième des mesures restant en n'activant des électrodes que à l'intérieur, sur les arêtes et/ou sur les coins de la face opposée.

Dans le cas où le système de détection équipe un système mobile 100 en rotation, la répartition du nombre de mesures effectuées dans les différentes directions de l'espace peut correspondre à celle décrite de manière simplifiée à deux dimensions à la figure 7 : dans ce cas, l'espace autour du système de détection est subdivisé en huit portions, quatre de ces portions étant explorées chacune pendant un cinquième du temps alloué aux mesures et les quatre autres (qui alternent avec les quatre premières) étant explorées chacune pendant un vingtième du temps alloué aux mesures.

Ces exemples ne sont pas limitatifs et la souplesse dans le choix du point de fonctionnement permet de créer simplement d'autres variantes.

Comme le boîtier de commutation permet de reconfigurer les électrodes de manière automatique suivant les consignes transmises par le bloc de génération de consignes, le système de détection peut dans un mode statique réaliser une cartographie au choix de tout ou partie de son environnement. Aucun mouvement du système de détection ou d'un système mobile 100 que le système de détection équipe n'est nécessaire pour obtenir la carte souhaitée, c'est-à-dire sonder les différentes directions d'intérêt, puisque la gestion de la configuration des électrodes, par exemple en tenant compte de résultats de mesure antérieurs, permet à elle seule de choisir la ou les directions explorées, ainsi que la distance maximale à laquelle ces directions sont explorées.

En mode portée, la distance maximale à laquelle la détection est possible dans au moins une direction donnée est optimisée.

L'amplitude et/ou la fréquence de chaque composante sinusoïdale du signal au niveau des électrodes dans l'état émettrice en mode portée sont déterminées en fonction de la nature du milieu conducteur.

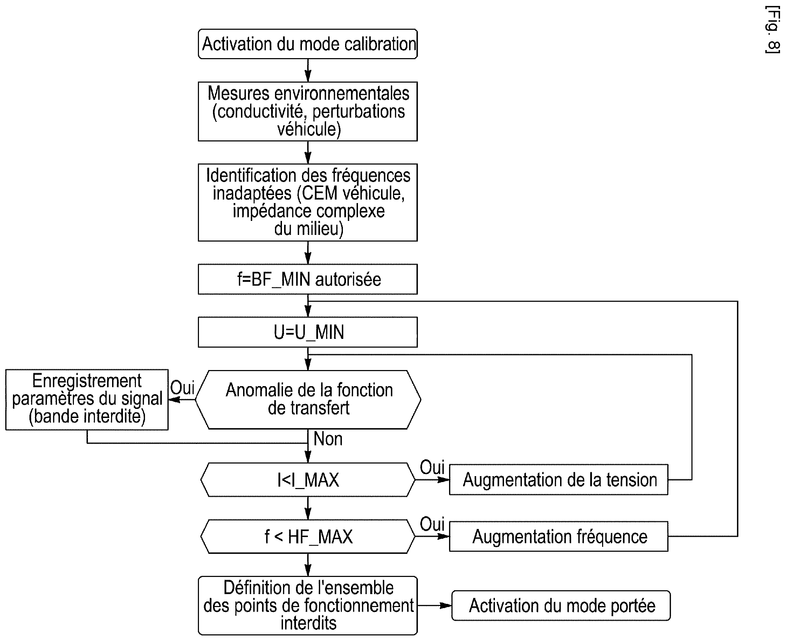

Une phase de calibration suivant l'algorithme de la figure 8 peut être envisagée avant la phase de mesure en mode portée. Il est pour cela possible d'effectuer un balayage en fréquence dans la gamme ] 0 Hz, 3 MHz] sur une répétition de n séries de mesures successives pour identifier la [les] fréquence[s] la [les] plus adaptée[s] pour maximiser la portée dans le milieu exploré. Dans l'eau, on peut par exemple choisir une fréquence de travail f = 10 kHz.

Cette phase de calibration peut aussi être exploitée pour identifier les fréquences de travail interdites, en particulier les fréquences propres (et leurs harmoniques) du système mobile 100 que le système de détection équipe. On inclut ici dans le système mobile 100 les équipements de ce système, par exemple un sonar.

Dans une réalisation particulière, le balayage en fréquence peut être effectué dans la gamme ] 0 Hz, 25 kHz], les basses fréquences étant le plus souvent les plus pertinentes pour optimiser la portée de détection.

Ces exemples ne sont bien sûr pas limitatifs : Il est possible de réaliser un balayage en fréquence dans la gamme ] 0 Hz, 3 MHz] pour déterminer la ou les fréquences de travail optimisant la portée de détection dans une direction donnée du milieu conducteur si par exemple la composition de ce dernier n'est pas connue a priori.

Si le système de détection est proche d'une interface eau/sédiments, le potentiel des électrodes émettrices pourra plus avantageusement être la composition de deux potentiels sinusoïdaux. Par exemple, on compose deux potentiels sinusoïdaux d'amplitude comprise entre 0 et 15 V et de fréquences dont l'une est égale à 10 kHz et l'autre supérieure à 10 kHz, par exemple 67 kHz. Le signal électrique de plus grande fréquence permet notamment d'obtenir une meilleure portée dans les sédiments. L'amplitude est fixée de manière à avoir les signaux les plus intenses possibles au niveau des électrodes réceptrices, sans dépasser la valeur seuil de l'intensité au niveau des électrodes en mode de mesure I.

Selon une réalisation, dans le procédé de détection, le point de fonctionnement du système peut correspondre à un mode « localisation », c'est-à-dire que la distance entre le système de détection et l'objet détecté dans une direction donnée est mesurée avec une précision maximale pour la valeur de distance particulière à laquelle se trouve l'objet, tout en restant dans un mode de fonctionnement nominal. Dans ce cas, les positions des électrodes placées dans les états émettrice ou réceptrice sont par exemple déterminées en fonction des directions dans lesquelles un objet a été détecté.

Dans un mode de réalisation particulier, l'amplitude et/ou la fréquence des composantes sinusoïdales du signal au niveau des électrodes dans l'état émettrice sont déterminées pour optimiser la précision de la localisation. L'amplitude du signal peut notamment être fixée en fonction de la position et de la nature de l'objet détecté ; en particulier la valeur maximale de l'amplitude qui permet, pour la position et la nature de l'objet détecté, de ne pas dépasser l'intensité maximale autorisée au niveau des électrodes réceptrices peut être choisie.

La fréquence du signal peut être choisie à l'issue d'un balayage en fréquence. Ainsi, lorsqu'un objet est présent dans le milieu conducteur, du fait de l'effet de peau, les signaux mesurés sont différents de ceux qui seraient mesurés en l'absence d'objet, si l'objet se trouve dans la zone de détection correspondant au point de fonctionnement choisi. A basse fréquence, la portée est plus grande qu'à haute fréquence mais la précision de la localisation est moins bonne. Quand la fréquence augmente, il existe donc une fréquence seuil au-delà de laquelle le signal mesuré est identique à celui du milieu conducteur sans objet. La fréquence de travail choisie pour la localisation est proche de la fréquence seuil, de manière à ce que la précision de la localisation soit maximale.

Ce mode de réalisation ne nécessite pas de déplacement du système de détection (ou d'un système mobile 100 qu'il équipe) par rapport à l'objet détecté, même si un tel déplacement reste possible. La reconfiguration - automatisée - des électrodes, et notamment de la fréquence du signal émis, suffit à obtenir les informations nécessaires pour l'optimisation de la localisation.

Selon une réalisation, le procédé de détection peut fixer le point de fonctionnement du système pour qu'il corresponde à un mode « identification », c'est-à-dire que la forme et/ou la nature de l'objet détecté sont déterminées avec une précision maximale.

Pour préciser la forme d'un objet d'intérêt, les électrodes placées dans les états émettrice ou réceptrice sont déterminées en fonction de la direction dans laquelle l'objet a été détecté et n séries de mesures sont effectuées de sorte que seules des électrodes à l'intérieur, sur les arêtes et/ou sur les coins de la face de normale sortante correspondant à la direction d'intérêt sont dans l'état connectée et que différentes combinaisons d'électrodes sont parcourues sur les n séries de mesures, de manière à déduire des mesures la forme de l'objet. Pour confirmer la forme de l'obstacle, les électrodes sont par exemple commutées de manière asymétrique d'une série de mesures à la suivante dans une direction donnée. On peut ainsi confirmer qu'un objet se trouvant sous la face inférieure du système de détection a une certaine extension suivant la direction (x'x) en connectant les électrodes de la face inférieure par groupes successifs suivant la direction (x'x), les autres électrodes de cette face étant déconnectées : d'abord les électrodes les plus proches de la face arrière puis leurs voisines dans la direction (x'x), jusqu'aux électrodes les plus proches de la face avant. Les différences ou analogies entre les résultats des n séries de mesures permettent alors de conclure sur la forme de l'objet dans la direction (x'x).

Pour confirmer la nature de l'obstacle, la fréquence de la tension électrique imposée aux électrodes dans l'état émettrice peut par exemple être modifiée pour effectuer un balayage dans la gamme ] 0 Hz, 3 MHz] sur une succession de n séries de mesures dans le mode « identification ».

Il est alors possible d'obtenir des informations sur la nature de l'objet détecté. Par exemple, la présence d'un objet isolant électrique se traduira par une intensité de courant électrique mesurée inférieure à l'intensité du courant mesurée en l'absence de cet objet, alors que si l'objet est conducteur de l'électricité, cette intensité sera supérieure à celle en l'absence de cet objet.

Un objet homogène de nature minérale n'induira par exemple pas de déphasage entre les signaux émis et les signaux mesurés alors que la présence d'un objet de nature biologique, dans la mesure où les cellules qui le constituent se comportent comme des condensateurs, se traduira par un déphasage entre les signaux émis et mesurés.

Il est aussi possible d'obtenir des informations sur un objet détecté (telles qu'à titre d'exemple la forme, taille, le caractère conducteur ou isolant électrique) de l'objet, par exemple par comparaison des mesures avec des références connues, c'est-à-dire une base de données d'objet et d'influence de l'immersion de cet objet dans le milieu conducteur sur les signaux électriques mesurés par le système de détection, soit sur la base de modèles d'évolution électriques et mécaniques de la scène, soit par comparaison des mesures avec des bases de signatures connues, soit par une information renseignée a priori dans le cadre de la mission. Les méthodes n'étant pas mutuellement exclusives.

Dans ce mode de fonctionnement, on peut ainsi constituer une base de données de signatures, dites signatures « sens électrique » d'objets d'intérêt, (tels que des mines, des câbles, des canalisations). Quand le système de détection est déployé in situ, on peut alors faire de la comparaison à la base de données pour en déduire une ou plusieurs informations sur un objet détecté.

La mise en œuvre du mode localisation ne nécessite pas (sans l'exclure !) de déplacement du système de détection (ou d'un système mobile qu'il équipe) par rapport à l'objet détecté. La reconfiguration - automatisée - des électrodes, et notamment de la fréquence du signal émis, suffit à obtenir les informations nécessaires pour l'identification.

Selon une réalisation, le procédé de détection peut être mis en œuvre au moyen d'un programme informatique exécuté sur un processeur intégré au système de détection. Les principales étapes de ce programme d'ordinateur vont maintenant être décrites en référence aux figures 8 à 11.

Dans un mode particulier de réalisation, le système de détection peut être placé au démarrage de la détection en mode « portée ». Un exemple d'algorithme détaillé pour le mode « portée » est donné à la figure 9. Une première étape facultative de balayage en fréquence, correspondant à une phase de calibration, peut être effectuée si le milieu n'est pas connu a priori, pour déterminer la fréquence de travail optimale dans ce mode.

En l'absence d'objet dans le milieu ou d'interface avec un autre milieu conducteur, l'impédance électrique du milieu prend une valeur de référence qui peut être donnée a priori ou mesurée lors de la phase de calibration. La présence d'un objet ou d'une interface modifie cette impédance électrique de sorte que la distance de l'objet ou de l'interface peut être évaluée.

Tant qu'aucun objet ou interface n'est détecté à une distance inférieure à une distance seuil d1, le système reste en mode portée. Si une interface avec un autre milieu est détectée à une distance inférieure à une distance seuil d1, le balayage en fréquence est renouvelé.

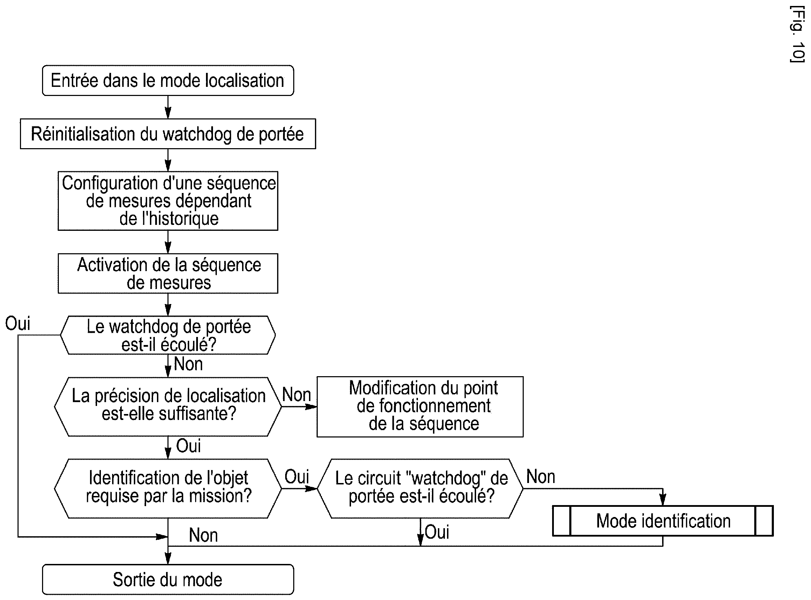

Sinon, si un objet est détecté à une distance seuil inférieure à une distance d2, le système est placé pour la mesure suivante en mode « localisation », dont un exemple d'algorithme est donné à la figure 10, et y reste tant que la distance entre l'objet et le système est supérieure à une distance seuil d3.

Si la distance entre l'objet et le système redevient supérieure à la distance d2, le système passe à nouveau en mode « portée ».

Si, au contraire, la distance entre l'objet et le système devient inférieure à la distance seuil d3 et si la mission le requiert, le système est placé pour la mesure suivante en mode « identification ». Il réalise alors des séries de mesures lui permettant de conclure sur la forme et la nature de l'objet avec la meilleure précision possible.

La mission peut être définie sous forme de consignes données a priori.

Lorsque l'identification est satisfaisante, la mission se poursuit avec le mode adapté à la tâche suivante.

Dans le cas où le système de détection équipe un système mobile, le mode « portée » peut être rendu prioritaire sur les autres modes pour éviter d'éventuelles collisions. Plus précisément, le système de détection contient un watchdog qui impose au système de détection de revenir au mode portée à chaque fois qu'une période du watchdog s'est écoulée.

Selon une réalisation, le procédé de détection peut être mis en œuvre sur un système mobile 100 dans le milieu conducteur d'électricité et équipé du système de détection. Les résultats du procédé de détection peuvent dans ce cas être utilisés pour guider le déplacement du système mobile 100, par exemple pour éviter des obstacles, ou bien positionner le système mobile à une distance et dans une orientation d'intérêt par rapport à une paroi, une interface ou un objet présent dans le milieu.

La mission peut donc intégrer des consignes en rapport avec le déplacement du système mobile 100.

Dans un mode de réalisation, le boîtier de configuration du point de fonctionnement se base sur l'historique des mesures pour déterminer le point de fonctionnement suivant.

Dans un autre mode de réalisation, le point de fonctionnement peut être fixé par un algorithme choisi a priori, indépendamment des mesures effectuées, dans la limite des seuils d'asservissement du système.

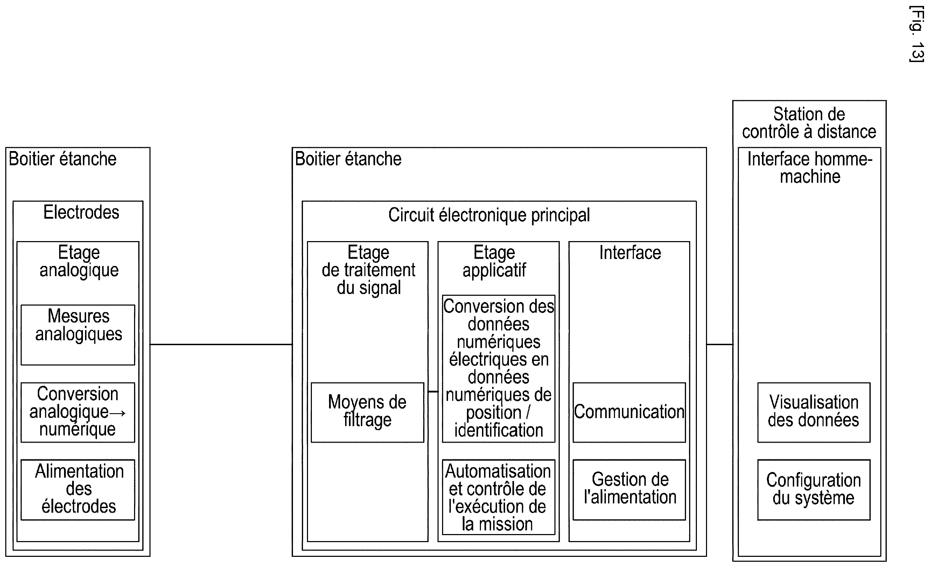

Dans un mode de réalisation particulier, dit « à électronique distribuée », le système de détection comprend un générateur par électrode et l'ensemble {générateur - électrode - moyens de mesure analogique - étage de conversion analogique/numérique} est relié par l'intermédiaire d'un faisceau de câbles souples au boîtier étanche contenant le bloc de génération de consigne et le boîtier de commutation, conformément à la figure 13. Ce mode de réalisation permet de ne faire transiter par les câbles souples que des signaux numériques, ce qui procure une meilleure immunité au bruit.

Il est aussi possible de ne prévoir qu'un seul générateur alimentant toutes les électrodes placées en mode connecté, le générateur se trouvant dans ce cas dans le boîtier étanche contenant le bloc de génération de consigne.

Enfin, dans un mode de réalisation particulier, le système de détection contient une interface de communication avec une station de contrôle à distance, par le biais de laquelle un opérateur peut visualiser les données issues du processus de détection et configurer le système de détection, notamment lui spécifier la ou les missions à accomplir.

Une fois le système de détection immergé, il est donc possible pour un opérateur distant de modifier la mission à distance ou encore de prendre la main sur le bloc de génération de consigne pour imposer une configuration particulière au système de détection.

Les multiples possibilités de configuration des électrodes du système de détection combiné avec le caractère automatique de la reconfiguration des électrodes, éventuellement avec l'intervention d'un opérateur distant, permettent donc au système de détection de fournir des données cartographiques de l'espace environnant sans connaissance a priori de cet espace. Les données cartographiques peuvent correspondre à une zone de l'espace environnant le système de détection qui peut être choisie de manière statique mais aussi évoluer de manière dynamique soit du fait du déplacement du système de détection, par exemple parce qu'il équipe un système mobile 100, soit du fait de résultats de détection antérieurs.

Par données cartographiques, on entend des informations ayant au moins un caractère spatial sur le milieu conducteur d'électricité. Il peut en particulier, mais de manière non limitative, s'agir de la position, et éventuellement de la forme d'éventuelles interfaces avec d'autres milieux (liquides, gazeux ou solide). Il peut aussi s'agir de la position (coordonnées spatiales) d'un objet solide dans ce milieu, et/ou de sa forme et/ou de la nature de cet objet, par exemple son caractère isolant ou conducteur électrique

Passer du mode portée au mode localisation par exemple revient à réduire la zone de l'espace couverte par la carte constituée pour se concentrer sur la zone dans laquelle un objet à été détecté, et éventuellement à changer l'échelle de la carte pour obtenir un niveau de détails plus important dans cette zone particulière.

### LISTE DES SIGNES DE REFERENCE

100 : système mobile équipé d'un système de détection
Ei (i entier de 1 à n) : i-ème électrode du système de détection
S1i (i entier de 1 à n) : interrupteur permettant de sélectionner l'état de l'électrode i si elle est connectée parmi les états émettrice et réceptrice.
S2i (i entier de 1 à n) : interrupteur permettant de sélectionner le mode de mesure de l'électrode i parmi les modes U et I.
S3i (i entier de 1 à n) : interrupteur permettant de sélectionner l'état de connexion de l'électrode i parmi les états connectée et déconnectée.

## Revendications

1. Procédé de détection dans un milieu conducteur d'électricité au moyen d'un système de détection,
le système de détection comportant :
- une pluralité d'électrodes (Eᵢ) en contact électrique direct avec ledit milieu, dont l'état peut être choisi dans la liste, émettrice, réceptrice, déconnectée,
- un dispositif de mesure d'au moins une grandeur électrique pour chacune desdites électrodes configurées dans l'état émettrice ou réceptrice, choisie dans la liste, intensité électrique traversant l'électrode, potentiel électrique de l'électrode,
- un dispositif de commutation permettant de placer chacune desdites électrodes dans l'état choisi dans la liste, émettrice, réceptrice, déconnectée,
- au moins un processeur échangeant des informations avec le dispositif de mesure et le dispositif de commutation,
le procédé comprenant les étapes :
a. le processeur détermine le point de fonctionnement du système de détection en fonction :
- d'une consigne donnée a priori,
- et/ou d'une configuration antérieure du système de détection,
- et/ou d'un résultat d'une mesure antérieure au niveau d'au moins une desdites électrodes transmis par le dispositif de mesure,
la détermination dudit point de fonctionnement du système de détection comprenant la détermination des trois paramètres suivants :
- état de chacune des électrodes, choisi parmi les trois états : émettrice, réceptrice, déconnectée,
- fréquence d'au moins une composante sinusoïdale d'un signal électrique émis par au moins une des électrodes configurées dans l'état émettrice,
- amplitude du signal électrique émis par au moins une des électrodes configurées dans l'état émettrice,
b. le dispositif de commutation reçoit des informations sur le point de fonctionnement du système déterminé par le processeur et configure le système de détection dans le point de fonctionnement déterminé,
c. une série de mesures est effectuée par le dispositif de mesure, une série de mesures consistant en l'évaluation d'au moins une grandeur électrique au niveau de chacune des électrodes configurées dans l'état réceptrice ou émettrice, et le dispositif de mesure transmet les données obtenues lors de la série de mesures au processeur.

2. Procédé de détection suivant la revendication 1 **caractérisé en ce qu'**il comprend en outre une étape supplémentaire, dite étape d, au cours de laquelle le processeur calcule à partir des données obtenues lors de la série de mesures au moins une donnée cartographique du milieu conducteur.

3. Procédé de détection dans un milieu conducteur d'électricité suivant la revendication 2 **caractérisé en ce que** si un objet est détecté à une étape d, les positions sur ledit système des électrodes configurées dans l'état émettrice pour un point de fonctionnement déterminé à une étape a ultérieure sont déterminées en fonction de la forme et/ou de la position d'un objet détecté.

4. Procédé de détection dans un milieu conducteur d'électricité suivant la revendication 2 ou la revendication 3 **caractérisé en ce que** des références connues sont utilisées pour déterminer l'au moins une donnée cartographique à l'étape d.

5. Procédé de détection suivant l'une quelconque des revendications 1 à 4 **caractérisé en ce que** les étapes du procédé sont répétées dans le même ordre au moins une fois, et **en ce que** des consignes sont transmises audit processeur pour contrôler la répétition des étapes soit avant la première étape a du procédé de détection par un opérateur distant ou non, soit au cours du procédé de détection par un opérateur distant.

6. Procédé de détection dans un milieu conducteur d'électricité suivant l'une des revendications 2 à 4 et la revendication 5 **caractérisé en ce que** si un objet est détecté à une étape d, l'amplitude et/ou la forme et/ou la fréquence des composantes sinusoïdales du signal électrique émis par chacune des électrodes configurées dans l'état émettrice pour un point de fonctionnement déterminé à une étape a ultérieure sont déterminées en fonction de la distance de l'objet détecté.

7. Procédé de détection dans un milieu conducteur d'électricité suivant l'une quelconque des revendications 1 à 6 **caractérisé en ce que** le point de fonctionnement du système déterminé à l'étape a peut être choisi dans la liste, mode « portée », mode « localisation », mode « identification, le mode, portée, permettant d'obtenir la portée de détection maximale dans une ou des directions données dudit milieu, le mode « localisation » permettant d'obtenir la précision maximale sur la localisation d'un objet antérieurement détecté, le mode « identification » permettant d'obtenir la meilleure résolution sur la forme et/ou la composition d'un objet antérieurement détecté.

8. Procédé de détection dans un milieu conducteur d'électricité suivant l'une des revendications 5 et 6 et selon la revendication 7, **caractérisé en ce que** le point de fonctionnement du système de détection passe automatiquement d'une étape a à la suivante :
- du mode « portée », s'il était dans ce mode, au mode « localisation » lorsqu'un objet est détecté et que cet objet est localisé à une distance inférieure à une distance seuil d2,
- du mode « localisation », s'il était dans ce mode, au mode « identification » lorsqu'un objet est détecté et que cet objet est localisé à une distance inférieure à une distance seuil d3 ou avec une forme et/ou une nature correspondant à une consigne,
- du mode « localisation », s'il était dans ce mode, au mode « portée» lorsqu'un objet est détecté et que la distance à laquelle se trouve un objet détecté devient supérieure à une distance seuil d2.

9. Procédé de détection dans un milieu conducteur d'électricité suivant l'une des revendications 1 à 8 **caractérisé en ce que** la forme et/ou la fréquence et/ou l'amplitude du signal émis par les électrodes configurées dans l'état émettrice pour un point de fonctionnement déterminé à une étape a sont choisies à l'issue d'un balayage en fréquence.

10. Procédé de détection dans un milieu conducteur d'électricité suivant l'une des revendications 1 à 9 **caractérisé en ce que** le signal émis par l'une au moins des électrodes configurées dans l'état émettrice pour un point de fonctionnement déterminé à une étape a est la composition d'au moins deux signaux sinusoïdaux de fréquences différentes.

11. Programme d'ordinateur comprenant des instructions de code de programme qui conduisent le système selon la revendication 12 à exécuter les étapes de la méthode selon l'une des revendications 1 - 10.

12. Système de détection dans un milieu conducteur d'électricité comprenant :
- une pluralité d'électrodes (Eᵢ) en contact électrique direct avec ledit milieu dont l'état peut être choisi dans la liste, émettrice, réceptrice, déconnectée,
- un dispositif de mesure d'au moins une grandeur électrique pour chacune desdites électrodes placées dans l'état émettrice ou réceptrice, choisie dans la liste {intensité du courant électrique traversant l'électrode, potentiel électrique de l'électrode},
- un dispositif de commutation permettant de placer chacune desdites électrodes dans l'état choisi dans la liste, émettrice, réceptrice, déconnectée,
- au moins un processeur échangeant des informations avec le dispositif de mesure et le dispositif de commutation, et configuré pour déterminer le point de fonctionnement du système de détection en fonction :
• d'une consigne donnée a priori,
• et/ou d'une configuration antérieure du système,
• et/ou d'un résultat d'une mesure antérieure au niveau d'au moins une desdites électrodes transmis par le dispositif de mesure,
la détermination dudit point de fonctionnement du système comprenant la détermination des trois paramètres suivants :
- état de chacune des électrodes, choisi parmi les au moins trois états : émettrice, réceptrice, déconnectée,
- fréquence d'au moins une composante sinusoïdale d'un signal électrique émis par au moins une des électrodes émettrices,
- amplitude du signal électrique émis par au moins une des électrodes configurées dans l'état émettrice,
le processeur étant configuré pour transmettre des informations sur le point de fonctionnement déterminé au dispositif de commutation.

13. Système mobile (100) dans un milieu conducteur d'électricité équipé d'un système de détection suivant la revendication 12 comportant en outre un module de contrôle adapté pour contrôler le déplacement dudit système mobile à partir des résultats de mesure du système de détection obtenues en suivant le procédé de l'une quelconque des revendications 1 à 10.

14. Système mobile dans un milieu conducteur d'électricité suivant la revendication 13 **caractérisé en ce que** lesdites électrodes du système de détection sont réparties sur au moins une partie de la surface dudit système mobile en contact avec ledit milieu.

## Patentansprüche

1. Verfahren zur Detektion in einem elektrisch leitfähigen Medium mittels eines Detektionssystems, wobei das Detektionssystem Folgendes aufweist:
- eine Vielzahl von Elektroden (E;) in direktem elektrischem Kontakt mit dem genannten Medium, wobei deren Zustand aus der Liste gewählt werden kann: Sendeelektrode, Empfangselektrode, abgekoppelt,
- eine Messeinrichtung zur Messung von mindestens einer elektrischen Größe für jede der genannten Elektroden, die in den Zustand Sendeelektrode oder Empfangselektrode konfiguriert sind, gewählt aus der Liste: elektrischer Strom durch die Elektrode, elektrisches Potenzial der Elektrode,
- eine Schaltvorrichtung, die es ermöglicht, jede der genannten Elektroden in den aus der Liste gewählten Zustand zu versetzen, Sendeelektrode, Empfangselektrode, abgekoppelt,
- mindestens ein Prozessor, der Informationen mit der Messeinrichtung und der Schaltvorrichtung austauscht,
Das Verfahren umfasst die folgenden Schritte:
a. Der Prozessor bestimmt den Betriebspunkt des Detektionssystems in Abhängigkeit von:
- einer vorab gegebenen Anweisung,
- und/oder einer vorherigen Konfiguration des Detektionssystems,
- und/oder eines Ergebnisses einer früheren Messung an mindestens einer der genannten Elektroden, das von der Messeinrichtung übermittelt wurde,
Die Bestimmung des genannten Betriebspunkts des Detektionssystems, einschließlich der Bestimmung der folgenden drei Parameter:
- Zustand jeder Elektrode, ausgewählt aus den drei Zuständen: Sendeelektrode, Empfangselektrode, abgekoppelt,
- Frequenz von mindestens einer sinusförmigen Komponente eines elektrischen Signals, das von mindestens einer der in den Zustand Sendeelektrode konfigurierten Elektroden ausgesendet wird,
- Amplitude des elektrischen Signals, das von mindestens einer der in den Zustand Sendeelektrode konfigurierten Elektroden ausgesendet wird,
b. Die Schaltvorrichtung erhält Informationen über den vom Prozessor bestimmten Betriebspunkt des Systems und konfiguriert das Detektionssystem auf den bestimmten Betriebspunkt,
c. Eine Messreihe wird von der Messeinrichtung durchgeführt, eine Messreihe besteht in der Auswertung von mindestens einer elektrischen Größe an jeder der Elektroden, die im Zustand Empfangselektrode oder Sendeelektrode konfiguriert sind, und dieMesseinrichtung übermittelt die bei der Messreihe erhaltenen Daten an den Prozessor.

2. Verfahren zur Detektion gemäß Anspruch 1, **dadurch gekennzeichnet, dass** es ferner einen zusätzlichen Schritt, bezeichnet als Schritt d, umfasst, während dessen der Prozessor aus den bei der Messreihe erhaltenen Daten mindestens eine kartografische Größe des elektrisch leitfähigen Mediums berechnet.

3. Verfahren zur Detektion in einem elektrisch leitfähigen Medium gemäß Anspruch 2, **dadurch gekennzeichnet, dass**, falls bei Schritt d ein Objekt detektiert wird, die Positionen auf besagtem System der Elektroden, die für einen bei einem späteren Schritt a bestimmten Betriebspunkt in den Zustand Sendeelektrode konfiguriert sind, entsprechend der Form und/oder der Lage des detektierten Objekts bestimmt werden.

4. Verfahren zur Detektion in einem elektrisch leitfähigen Medium gemäß Anspruch 2 oder Anspruch 3, **dadurch gekennzeichnet, dass** bekannte Referenzen verwendet werden, um die mindestens eine kartografische Größe in Schritt d zu bestimmen.

5. Verfahren zur Detektion gemäß einem der Ansprüche 1 bis 4, **dadurch gekennzeichnet, dass** die Verfahrensschritte mindestens einmal in derselben Reihenfolge wiederholt werden und dass Anweisungen an den genannten Prozessor übermittelt werden, um die Wiederholung der Schritte zu steuern, entweder vor dem ersten Schritt a des Detektionsverfahrens durch einen entfernten oder örtlichen Bediener, oder während des Detektionsverfahrens durch einen entfernten Bediener.

6. Verfahren zur Detektion in einem elektrisch leitfähigen Medium gemäß einem der Ansprüche 2 bis 4 und Anspruch 5, **dadurch gekennzeichnet, dass**, wenn ein Objekt in einer Stufe d detektiert wird, die Amplitude und/oder die Form und/oder die Frequenz der sinusförmigen Komponenten des von jeder als Sendeelektrode konfigurierten Elektrode für einen in einer späteren Stufe a bestimmten Betriebspunkt ausgesendeten elektrischen Signals in Abhängigkeit von der Entfernung des detektierten Objekts bestimmt werden.

7. Verfahren zur Detektion in einem elektrisch leitfähigen Medium gemäß einem der Ansprüche 1 bis 6, **dadurch gekennzeichnet, dass** der in Schritt a bestimmte Betriebspunkt des Systems aus der Liste gewählt werden kann: Modus «Reichweite», Modus «Ortung», Modus «Identifizierung», wobei der Modus «Reichweite» die maximale Detektionsreichweite in einer oder mehreren gegebenen Richtungen des genannten Mediums ermöglicht, der Modus «Ortung» die maximale Genauigkeit bei der Ortung eines zuvor detektierten Objekts ermöglicht und der Modus «Identifizierung» die beste Auflösung bezüglich Form und/oder Zusammensetzung eines zuvor detektierten Objekts ermöglicht.

8. Verfahren zur Detektion in einem elektrisch leitfähigen Medium gemäß einem der Ansprüche 5 und 6 und nach Anspruch 7, **dadurch gekennzeichnet, dass** der Betriebspunkt des Detektionssystems automatisch von einer Stufe a zur nächsten übergeht:
- vom Modus «Reichweite», falls er sich in diesem Modus befand, in den Modus «Ortung», wenn ein Objekt detektiert wird und dieses Objekt in einer Entfernung kleiner als einer Schwellendistanz d2 lokalisiert ist,
- vom Modus «Ortung», falls er sich in diesem Modus befand, in den Modus «Identifizierung», wenn ein Objekt detektiert wird und dieses Objekt in einer Entfernung kleiner als einer Schwellendistanz d3 lokalisiert ist oder eine Form und/oder Beschaffenheit aufweist, die einer Anweisung entspricht,
- vom Modus «Ortung», falls er sich in diesem Modus befand, in den Modus «Reichweite», wenn ein Objekt detektiert wird und der Abstand des detektierten Objekts größer als eine Schwellendistanz d2 wird.

9. Verfahren zur Detektion in einem elektrisch leitfähigen Medium gemäß einem der Ansprüche 1 bis 8, **dadurch gekennzeichnet, dass** die Form und/oder die Frequenz und/oder die Amplitude des von den als Sendeelektroden konfigurierten Elektroden für einen in Schritt a bestimmten Betriebspunkt ausgesendeten Signals nach einem Frequenzscan gewählt werden.

10. Verfahren zur Detektion in einem elektrisch leitfähigen Medium gemäß einem der Ansprüche 1 bis 9, **dadurch gekennzeichnet, dass** das von mindestens einer der als Sendeelektroden konfigurierten Elektroden für einen in Schritt a bestimmten Betriebspunkt ausgesendete Signal aus mindestens zwei sinusförmigen Signalen unterschiedlicher Frequenzen besteht.

11. Computerprogramm, das Programmcodeanweisungen enthält, die das System gemäß Anspruch 12 veranlassen, die Schritte des Verfahrens nach einem der Ansprüche 1-10 auszuführen.

12. Detektionssystem in einem elektrisch leitfähigen Medium, umfassend:
- eine Vielzahl von Elektroden (Eᵢ) in direktem elektrischem Kontakt mit dem genannten Medium, deren Zustand aus der Liste gewählt werden kann: Sendeelektrode, Empfangselektrode, abgekoppelt,
- eine Messeinrichtung zur Messung mindestens einer elektrischen Größe für jede der genannten Elektroden, die in den Zustand Sendeelektrode oder Empfangselektrode versetzt sind, gewählt aus der Liste {elektrischer Strom durch die Elektrode, elektrisches Potenzial der Elektrode},
- eine Schaltvorrichtung, die es ermöglicht, jede der genannten Elektroden in den aus der Liste gewählten Zustand zu versetzen: Sendeelektrode, Empfangselektrode, abgekoppelt,
- mindestens ein Prozessor, der Informationen mit der Messeinrichtung und der Schaltvorrichtung austauscht und so konfiguriert ist, den Betriebspunkt des Detektionssystems in Abhängigkeit von:
• einer zuvor vorgegebenen Anweisung,
• und/oder einer vorherigen Konfiguration des Systems,
• und/oder eines Ergebnisses einer vorangegangenen Messung an mindestens einer der genannten Elektroden, das von der Messeinrichtung übermittelt wurde,
Die Bestimmung des genannten Betriebspunkts des Detektionssystems umfasst die Bestimmung der folgenden drei Parameter:
- Zustand jeder Elektrode, gewählt aus mindestens drei Zuständen: Sendeelektrode, Empfangselektrode, abgekoppelt,
- die Frequenz mindestens einer sinusförmigen Komponente eines von mindestens einer der Sendeelektroden ausgesandten elektrischen Signals,
- Amplitude des von mindestens einer der als Sendeelektrode konfigurierten Elektroden ausgesendeten elektrischen Signals,
der Prozessor ist so konfiguriert, Informationen über den bestimmten Betriebspunkt an die Schaltvorrichtung zu übermitteln.

13. mobiles System (100) in einem elektrisch leitfähigen Medium, ausgestattet mit einem Detektionssystem nach Anspruch 12, das ferner ein Steuerungsmodul aufweist, das eingerichtet ist, die Bewegung des genannten mobilen Systems anhand der mittels des Verfahrens eines der Ansprüche 1 bis 10 erhaltenen Messergebnisse des Detektionssystems zu steuern.

14. Mobiles System in einem elektrisch leitfähigen Medium nach Anspruch 13, **dadurch gekennzeichnet, dass** die genannten Elektroden des Detektionssystems auf mindestens einem Teil der Oberfläche des genannten mobilen Systems verteilt sind, der mit dem genannten Medium in Kontakt steht.

## Claims

1. A method for detecting electrical conductivity in a medium using a detection system, the detection system comprising: - a plurality of electrodes (Ei) in direct electrical contact with said medium, the state of which can be selected from the list emitting, receiving, disconnected, - a measuring device for at least one electrical quantity for each of said electrodes configured in the emitting or receiving state, selected from the list electrical current through the electrode, electrical potential of the electrode, - a switching device allowing each of said electrodes to be placed in the state selected from the list emitting, receiving, disconnected, - at least one processor exchanging information with the measuring device and the switching device, the method comprising the steps: a. The processor determines the operating point of the detection system based on: - a predefined setpoint, - and/or a previous configuration of the detection system, - and/or the result of a previous measurement at at least one of the electrodes transmitted by the measuring device. The determination of said operating point of the detection system includes determining the following three parameters: - the state of each electrode, chosen from the three states: transmitting, receiving, disconnected, - the frequency of at least one sinusoidal component of an electrical signal emitted by at least one of the electrodes configured in the transmitting state, - the amplitude of the electrical signal emitted by at least one of the electrodes configured in the transmitting state. b. The switching device receives information on the system's operating point determined by the processor and configures the detection system to the determined operating point. c. A series of measurements is performed by the measuring device, consisting of the evaluation of at least one electrical quantity at each of the electrodes configured in the receiving or transmitting state, and the measuring device transmits the data obtained during the series of measurements to the processor.

2. A detection method according to claim 1, **characterized in that** it further comprises an additional step, referred to as step d, during which the processor calculates, from the data obtained during the series of measurements, at least one mapping data point of the conductive medium.

3. A detection method in an electrically conductive medium according to claim 2, **characterized in that** if an object is detected at step d, the positions on said system of the electrodes configured in the transmitting state for a given operating point at a subsequent step a are determined based on the shape and/or position of the detected object.

4. A detection method in an electrically conductive medium according to claim 2 or claim 3, **characterized in that** known references are used to determine at least one map data point at step d.

5. A detection method according to any one of claims 1 to 4, **characterized in that** the steps of the method are repeated in the same order at least once, and **in that** instructions are transmitted to said processor to control the repetition of the steps either before the first step a of the detection method by a remote or non-remote operator, or during the detection method by a remote operator.

6. A detection method in an electrically conductive medium according to any one of claims 2 to 4 and claim 5, **characterized in that** if an object is detected at step d, the amplitude and/or shape and/or frequency of the sinusoidal components of the electrical signal emitted by each of the electrodes configured in the transmitting state for a specified operating point at a subsequent step a are determined as a function of the distance to the detected object.

7. A detection method in an electrically conductive medium according to any one of claims 1 to 6, **characterized in that** the operating point of the system determined in step a can be selected from the list of modes: "range," "localization," and "identification", wherein the "range" mode allows for obtaining the maximum detection range in one or more given directions of said medium, the "localization" mode allows for obtaining maximum accuracy in locating a previously detected object, and the "identification" mode allows for obtaining the best resolution regarding the shape and/or composition of a previously detected object.

8. A detection method in an electrically conductive medium according to any one of claims 5 and 6 and according to claim 7, **characterized in that** the operating point of the detection system automatically transitions from one step a to the next:
- from the "range" mode, if it was **in that** mode, to the "localization" mode " when an object is detected and located at a distance less than a threshold distance d2;
- from "Location" mode, if it was in this mode, to "Identification" mode when an object is detected and located at a distance less than a threshold distance d3 or with a shape and/or nature corresponding to a setpoint;
- from "Location" mode, if it was in this mode, to "Range" mode when an object is detected and the distance to the detected object becomes greater than a threshold distance d2.

9. A detection method in an electrically conductive medium according to any one of claims 1 to 8, **characterized in that** the shape and/or frequency and/or amplitude of the signal emitted by the electrodes configured in the transmitting state for a determined operating point at a step a are chosen following a frequency sweep.

10. A detection method in an electrically conductive medium according to any one of claims 1 to 9, **characterized in that** the signal emitted by at least one of the electrodes configured in the transmitting state for a specified operating point at a step a is the composition of at least two sinusoidal signals of different frequencies.

11. A computer program comprising program code instructions for executing the steps of the method according to any one of claims 1 to 10 when said program is executed on a computer.

12. A detection system in an electrically conductive medium comprising:
- a plurality of electrodes (Ei) in direct electrical contact with said medium, the state of which can be selected from the list: emitting, receiving, disconnected;
- a measuring device for at least one electrical quantity for each of said electrodes placed in the emitting or receiving state, selected from the list: current intensity through the electrode, electrical potential of the electrode;
- a switching device allowing each of said electrodes to be placed in the state selected from the list: emitting, receiving, disconnected;
- at least one processor exchanging information with the measuring device and the switching device, and configured to determine the operating point of the detection system based on:
• a predetermined setpoint,
• and/or a previous system configuration,
• and/or a result of a previous measurement at at least one of said electrodes transmitted by the measuring device,
the determination said operating point of the system comprising the determination of the following three parameters:
- state of each of the electrodes, chosen from at least three states: transmitting, receiving, disconnected,
- frequency of at least one sinusoidal component of an electrical signal emitted by at least one of the transmitting electrodes,
- amplitude of the electrical signal emitted by at least one of the electrodes configured in the transmitting state, the processor being configured to transmit information on the determined operating point to the switching device.

13. A mobile system (100) in an electrically conductive medium equipped with a detection system according to claim 12, further comprising a control module adapted to control the movement of said mobile system based on the measurement results of the detection system obtained by following the method of any one of claims 1 to 10.

14. A mobile system in an electrically conductive medium according to claim 13, **characterized in that** said electrodes of the detection system are distributed over at least a portion of the surface of said mobile system in contact with said medium.
